# EUROPEAN PATENT APPLICATION

(11) **EP 3 088 509 A1**
(43) Date of publication of application: **02.11.2016**
(21) Application number: 15461536.3
(22) Date of filing: 22.05.2015
(51) Int. Cl.: C12M 1/02, C12M 1/00

(54) **DUAL-CHAMBER REACTOR FOR MAGNETIC ASSISTING OF CHEMICAL PROCESSES AND THE SYSTEM OF THIS REACTOR**

(30) Priority: 30.04.2015 PL 41217415
(71) Applicant: Zachodniopomorski Uniwersytet Technologiczny w Szczecinie, 70-310 Szczecin (PL)
(72) Inventor: Rakoczy, Rafal, 70-215 Szczecin (PL); Kordas, Marian, 74-510 Trzcinsko- Zdrój (PL); Fijalkowski, Karol, 71-221 Szczecin (PL); Konopacki, Maciej, 70-250 Szczecin (PL); Zywicka, Anna, 73-150 Lobez (PL); Peitler, Dorota, 83-300 Lebork (PL)
(74) Representative: Zawadzka, Renata

(57) **Abstract**

The subject of the invention is a dual-chamber reactor for magnetic assisting of the chemical processes involving gases, liquids and solids, and chemical or biochemical reactions carried out by microorganisms equipped with two mixing chambers, the generator of rotating magnetic field, a housing with a top cover and a bottom with connecting pieces, characterized in that, it has a central cylindrical partition (2), which forms inside the reactor (1), the two, cylindrical (3) and an annular (4) chambers fixed to the cone-shaped bottom (5). The reactor has an outer cylindrical housing (6), wherein the generator (7) of the rotating magnetic field is placed, located between the top cover (8) and cone-shaped bottom (5). The outer cylindrical housing (6) is equipped with inlet pipe (9) and outlet pipe (10) with the thermal medium. The cone-shaped bottom (5) has a central outlet pipe (11), a top cover (8) has inlet pipe (12), for supplying additional chemicals into the reactor. Reactor (1), under the generator (7) has at least one inlet pipe (13) for the reaction medium.

The system of reactor described above, is characterized in that the central outlet pipe (11) of the reactor (1) is connected to the inlet of the tank (28), which outlet is connected to the first circulation pump (30), wherein the tank (28) has a heating jacket (31).

## Description

The subject of the invention is a dual-chamber reactor for magnetic assisting of the chemical processes involving gases, liquids and solids, and chemical or biochemical reactions carried out by microorganisms and also including system of this reactor.

Bio-processes are carried out in systems characterized by high energy consumption, which requires incurring of significant costs. Developed biotechnologies require conducting of processes with the participation of living matter in an intermittent manner, while the economics and technical conditions are usually associated with the necessity of the system running in continuous operation. Typically, commercial processes of microorganisms reproduction are carried out in a batch or less frequently in semi-batch methods. Culturing of microorganisms or conducting of bioprocesses in continuous manner are particular difficult because of the problems connected with the sufficient process control and ensurement of an appropriate level of purification of the culture medium from adverse substances. Obtaining a satisfactory degree of purification of bio-product involves the use of an appropriate thermal treatment, and ensuring the stability of process parameters. An additional disadvantage of running the processes using microorganisms in a continuous manner is too short residence of living matter and/or reagents in the bioreactor, resulting from incorrect geometrical construction of culture apparatus.

In the literature, many methods are described on performance of chemical processes and bioprocesses, however there is a need to improve it in order to optimize processes for the production of bio-products, taking into account the economic aspects and the scale of production.

From the Patent Application US 2012/0100572 the invention concerning station containing a bioreactor equipped with cylindrical tank and jet agitator is known. Proper placement of jet nozzle ensure incorporating of living matter into a liquid medium and mixing of cell culture media, as well as providing the necessary nutrients or the media. The solution allows to increase the speed of processes conducted using micro-organisms and intensify mass transport processes in mixed media. Mixing application using the jet allows for a uniform level of dispersion of gases in the mixed medium and avoiding disintegration of living matter.

Description of the invention application CA 2727665 concerns mixing apparatus used for the suspension of solute in a solvent using magnetic core as a power source which actuate fluid in motion. The mixing process is triggered by electromagnetic fields produced out of the tank. Using this magnetic field allows to equalize the temperature and concentration gradients in a mixed fluid resulting in the acceleration of the kinetics of the dissolution process.

Known from Patent Application CN 101655054 mixer with the static magnetic field is used to support fuel combustion process in the combustion engine. A strong magnetic field can increase the effect of the mixing process in two-phase systems such gas-gas, gas-liquid, liquid-liquid and increases the combustion rate by increasing the process of providing oxygen to the combusted fuel. This results in improving the engine power and saves a considerable amount of fuel.

Presented in the Patent Application CN 102847477 magnetic stirring device consists of a winding (constructed of three pairs of Helmholtz coils and a pair of Maxwell coils), power supply and solid nanoparticles. The winding system generates gradient magnetic field in different time sequences, which is associated with the movement of solid particles with magnetic properties in a mixed medium.

In the description of Utility Model Application CN 103240016, mixing device operating in a continuous manner is presented. The mixer consists of a horizontally section of a pipe, wherein axially shaft is placed with the spaced systems of impellers. Windings are placed on the outer side of the pipe, so that the generated field covered working area of the blades. Area of the influence of agitator in liquid is in direct impact of magnetic field induced by the current flowing through the coil phase. The applied rotating magnetic field drives stirrers arranged on the shaft, generating areas of increased intensity of turbulence, which occurrence allows for acceleration of the rate of reaction.

Description of the utility model application CN 202157075 concerns an air-lift bioreactor with an external magnetic field generator. The apparatus consists of a flow bioreactor with an internal circulation of fermentation broth, equipped with inlet and outlet pipes. The outlet pipe is connected to the pipeline, on which magnetic field generator, a heat exchanger (for conditioning of the temperature of the culture medium), and circulation pump are mounted. The use of stationary and variable magnetic fields can improve the dissolution of oxygen in the culture medium in order to improve the process conditions in the tank bioreactor.

An application of the utility model CN 202576087 describes a bioreactor with magneto-electric induction for conducting the process of ammonia oxidation carried out by anaerobic bacteria (anammox process). Multi-sectional device is equipped with a system generating a magnetic field, electric field and the control system. The apparatus is used to activate the bacteria which are characterized by reduced biological activity or are in the inactive phase. The use of this type of physical interactions can increase the biological activity of living matter and the intensification of the enzymatic processes.

Description of the utility model application CN 203212576 relates to an apparatus with magneto-fluidization applied for the fermentation process and the preparation of extracts used in the production of flavored cigarettes. The apparatus consists of a reactor tank filled with microcarriers, characterized by magnetic susceptibility, which serve to immobilize living matter, enzymes and bioactivators. The application of the magnetic field can extend the working volume of the reactor and to avoid problems in conventional bioreactors, involving the process of mass transfer and performance of catalytic reactions carried out with the participation of living matter.

From the international application WO 2011/112601 a method of continuous culture of cells and cell products using a magnetically stabilized tissue culture is known. For the culture, the flow column apparatus divided into three sections: an inlet chamber, a part of the external application of magnetic field (magnetic field is generated by a ring made of neodymium) and the chamber for the biomass growth are used. The strong magnetic field allows for the spatial impacts on cultivated biological material and the use of magnetic carriers for the immobilization of enzymes or the process of magnetic separation.

Description of the Patent Application DE 102004026448 relates to a mixer equipped with a permanent magnet and the magnetic particles used for the immobilization of biological material or enzymes. The reactor may be applied in the hydrogenation reaction, catalytic reaction (the catalyst is in this case immobilized on magnetic media); conversion processes performed in the biomedical tasks, biological, pharmaceutical, foodstuffs; ion exchange reactions; polymerization reactions.

Patent EP 391 846 describes apparatus for cells perfusion and their culture in suspension. The apparatus consists of a tank containing a central zone for the cell culturing and the outer zone, arranged peripherally. The two zones are separated by a partition in order to ensure adequate circulation of the culture medium and the medium in the reactor. The flowing stream of the culture medium, by suitable configuration of partitions allows for circulation of microorganisms between the conical zone and the deposition zone in which the culture process is performed.

From the description of Patent Application EP 2039754 a tank bioreactor equipped with a stack of turbine agitators and bubbler of special design, used to provide uniform distribution of concentration in the mixed medium, is known. A suitable geometrical configuration of the mixer allows to control oxygen transfer coefficients in the upper and the lower part of the reactor.

Description of the U.S. Patent 5342781 defines the construction of air-lift bioreactor with an outer-loop circulation for culturing microorganisms, plant and animal cells, for obtaining valuable metabolites. The tube is equipped with a chamber in the shape of a truncated cone, creating a drop-zone. On the inner walls of the cone lamellar surfaces are placed in order to increase the separation efficiency of the cells of living matter from the culture medium.

In the description of Patent Application US 4892818, bioreactor system consisting of vertical columns divided into two sections and working in a continuous regime, is shown. Upper and lower sections are connected to the truncated cone in the way that the top section has a diameter greater than the lower section. The lower part of the apparatus is filled with particles designed to intensified biomass growth. The top section is equipped with a stack of mixers in order to increase the yield of the reaction and purification of the product.

The Patent Application No. 4906577 presents apparatus for cell culturing, in particular to processes in which living matter is vulnerable to shear. The apparatus consists of a culture chamber and placed above culture conditioning chamber. The growth chamber is placed coaxially in tube in which helical agitator works. In addition, to that part of the device, a gas is connected using a pipe.

Description of the Patent US 5248613 presents a bioreactor constructed from a cylindrical conduit in which the internal circulation pipe is placed coaxially. In the upper part of the apparatus truncated cone is placed in order to isolate the contact zone between bio liquid and gas.

Description of the U.S. Patent 5403742 relates to a fixed film bioreactor consisting of a cylindrical vessel in which a rotating shaft is placed coaxially. To the stirrer shaft four rectangular screens for immobilizing of microorganisms are fixed.

The description of the Patent Application US 5728577 defines a bioreactor equipped with a rotating shaft attached to its structure, on which the element for immobilization of microorganisms are placed. The proposed design solution provides more efficient mass transport between microorganisms and the culture medium enriched with gas.

The Patent PL 198449 describes a process for treating biological processable substances using microorganisms disposed on media. Biomass is produced using a reactor consisting of an upper distribution chamber, the lower bioreactor chamber placed under the distribution chamber and parts supplying biological recyclable materials to the distribution chamber. The modular design of the apparatus does not require mechanical aeration and works in continuous regime, which allows for a significant reduction in operating costs.

The polish Patent application No. 394335 describes a magnetic stirrer comprising a reservoir, external electric winding and a system of mixers. The apparatus is provided with internal electrical wiring arranged on the magnetic diffusers mounted coaxially in the tank and vertical divisions with turbines arranged at the wall of the tank. Produced by three-phase electric winding rotating magnetic field power the stirrer (made of a material susceptible to this type of interaction), which causes circulation of the liquid inside the camera.

Considering the above-described state of the art the following observations can be seen regarding the production of biomass using a reactor equipped with a mechanical stirrers and reactors and that employ physical interactions such as an eclectic field and / or magnetic fields used for chemical processes involving living matter: i) obtained bioproducts must be produced in the quickest and easiest way; ii) in the bio process on an industrial scale cheap medium (readily available industrial substrate) while maintaining a good quality of the product, should be used; iii) costly technological solutions should be avoided; iv) bioproducts should be readily available in the form of a pure culture; v) bioproducts should also be genetically stable; vi) developed bioreactor should be ease for scale transfer.

Taking into account the above comments, it is advisable to develop a reactor to intensify biomass production. The main object of the invention is to develop a dual chamber magnetically assisted reactor operating in a continuous regime capable for achieving the economic benefits as a reducing cost of the production.

The dual-chamber magnetically assisted reactor for processing in different phases of gas, liquid and solid states, such as chemical reactions or biochemical processes with the application of the living microorganisms is equipped with two mixing chamber, generator of rotating magnetic field, housing, top cover and bottom with connector pipes. According to the invention, the reactor is characterized by a central cylindrical partition which forms cylindrical and annular chambers inside the reactor attached to the cone-shaped bottom. The application of the cylindrical partition enable flowing of the medium (e.g. culture medium), upward through the annular chamber (the riser) and downward through the cylindrical chamber (the downcomer). The reactor has an external cylindrical housing, wherein the generator of rotating magnetic field is located, between the cover and the cone-shaped bottom. The external cylindrical housing has inlet and outlet pipes for heating the medium (the housing is filled with a cooling medium in order to cool a reactor), so that there is a jacket which provides a stable temperature conditions for a medium flowing through the reactor. The cylindrical part of reactor is connected with the cone-shaped bottom and top cover by using a flange connection. The application of this type of connection allows to manufacture the apparatus in sections and to seal a reactor. In the lower cylindrical part of reactor, under the generator of rotating magnetic field at least one pipe is placed in order to supply the reaction medium. This medium, in the case of bioprocessing comprises microorganisms. The cone-shaped bottom is equipped with a central outlet pipe. On the top cover a pipe is placed to supply the additional chemicals into the reactor.

As the generator of rotating magnetic field a stator of squirrel-cage induction motor is used and the stator windings are powered by the three-phase current. By using this type of current, it is possible to obtain a rotating magnetic field (in the area inside the stator) characterizing by heterogeneity of the magnetic induction values and its stability at time.

The inner diameter of the magnetic field generator is preferably similar to the outer diameter of the outer pipe. The gap between the inner diameter of the stator and the outer diameter of the cylindrical pipe is preferably minimized. In the outer cylindrical housing, between the generator and annular chamber a liquid with magneto- or electrorheological properties can be used, in order to increase the effect of magnetic field on the reactor chamber generated by the stator windings and to homogenize and increase the magnetic induction values obtained within the stator.

Preferably, in the annular chamber of the reactor above the inlet pipes at least two vertical cylindrical partitions are placed, wherein the vertical cylindrical partitions are arranged at equal distances to each other in a circle. Each partition is equipped with a sparger in order to supply the process gas.

Another version of the reactor is characterized by the annular chamber, wherein above the inlet pipes a toroidal sparger is preferably applied.

The reactor with toroidal sparger is preferably equipped with a turbine impeller which is placed in the cylindrical chamber and the upper part of the cylindrical partition has perforation. The shaft of the turbine impeller is introduced by the central pipe in the top cover and is connected with the drive enabling the reciprocating and/or rotational movement. The reactor with toroidal sparger is preferably equipped with a reciprocating piston in the cylindrical chamber and the lower part of the cylindrical partition has a perforation. The shaft of the piston is introduced by the central pipe in the top cover and is connected with drive enabling the reciprocating movement.

In the cylindrical and annual chambers of the reactor cylindrical and annual packings made from the material which is susceptible to a magnetic field are preferable placed. Packings are mounted to the shaft using rods and to the central cylindrical partition, which is in the form of two cylinders wherein is a slide bearing. The shaft is equipped with a bearing and is introduced by the central pipe in the top cover and connected with drive enabling the rotational movement.

The outer cylindrical housing is preferably connected with a heat exchanger by pipes, which ensure stable working conditions of the generator. The heat exchanger is preferably applied with a counter-flow lamella heat exchanger. As a circulating medium liquid characterizing by a high heat capacity is preferably used. The flange of top cover of reactor is indirectly connected with the flange of the cooling jacket for the winding by a loose flange with the appropriate seals. This connection provides a seal to both, the interior of the reactor and the cooling chamber for winding and the problem-free access to the winding if it should be replaced or control. Between the outlet pipe and the heat exchanger the first circulating pump is placed.

The top cover has preferably a pipe for measuring and controlling the apparatus. This cover can be equipped with a pipe in order to install safety and vent values, feeding pipes for additional chemicals into the reactor.

The cone-shaped bottom has preferably a lateral connector pipe which allows to remove medium from reactor, and particularly the medium located in the annular chamber.

According to the invention, the system with the described above reactor is characterized, by the central outlet of reactor which is connected with the inlet of tank, of which outlet is connected with the second circulation pump. The tank has a heating jacket. This tank may be treated as a storage tank for the obtained product or as a dump tank for the fluid in a bioreactor. The effluent stream from the rector can be directed back to the storage tank or in part to the inlet of the apparatus in order to circulate medium in the rector and more efficient use of medium. The circulation system of receiving heat medium or transferring heat medium is coupled with a temperature stabilization system for the storage or dump tank.

The advantage of this solution is that the technological process is carried out in the dual-chamber reactor, which is assisted by the rotating magnetic field, wherein this process can be realized in the batch, semi-continuous or continuous manner. The system of magnetically assisted processes consists of reactor, storage tank with heating jacket, dump tank, heat exchanger and a set of circulating pumps for liquid.

The division of the reaction chamber into two zones (annular and cylindrical chambers) allows to extend residence time of the medium in the reactor in order to carry out the process in the continuous manner. The separation of the inner reaction located in the inner tube allows to apply the various types of packings in order to immobilize the various types of biocatalysts as well as the application of the mixing systems in order to intensify the transport processes in the reaction mixture. The use of this type of the constructional solution allows to increase the area of interfacial contact and to homogenize temperature profiles inside the apparatus.

The invention allows for the realization of the processes in the continuous manner and to significant increase the residence time distribution of medium in the reaction unit as a result of the separation of the reaction zone into two, cylindrical and annular chambers. This separation allows the use of the additional constructional solutions, included in examples, which significantly influence on the yield of the obtained product. The advantage of the proposed reactor is to use the rotating magnetic field, which impacts on the realized chemical processes or bioprocesses with the participation of the living matter. In the case of the process realized with the participation of the living matter, the main advantage of the effect of the rotating magnetic field is the intensification of the physical mass transport, momentum and energy between the medium and the microorganisms or the direct effect of this kind of physical effect on microorganism, which influences on the quality and quantity of the biomass. The proposed solution provides the heat recovery and its use in maintaining the correct temperature of the medium with the microorganisms.

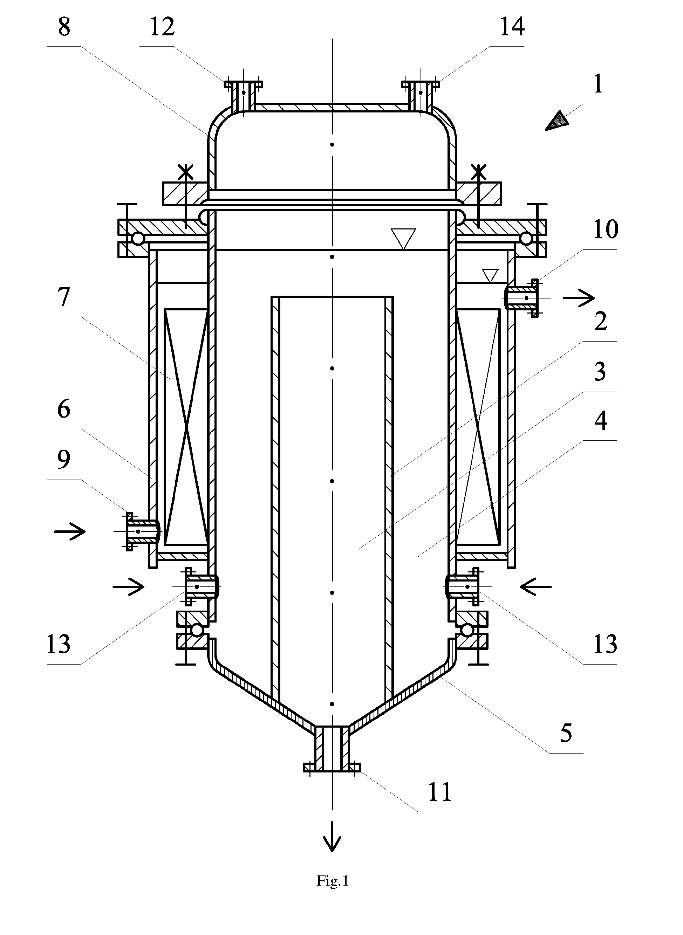

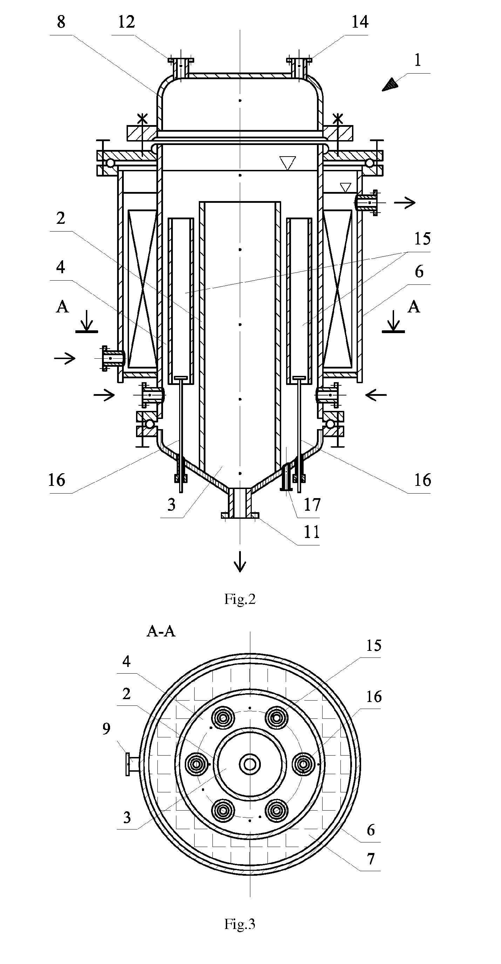

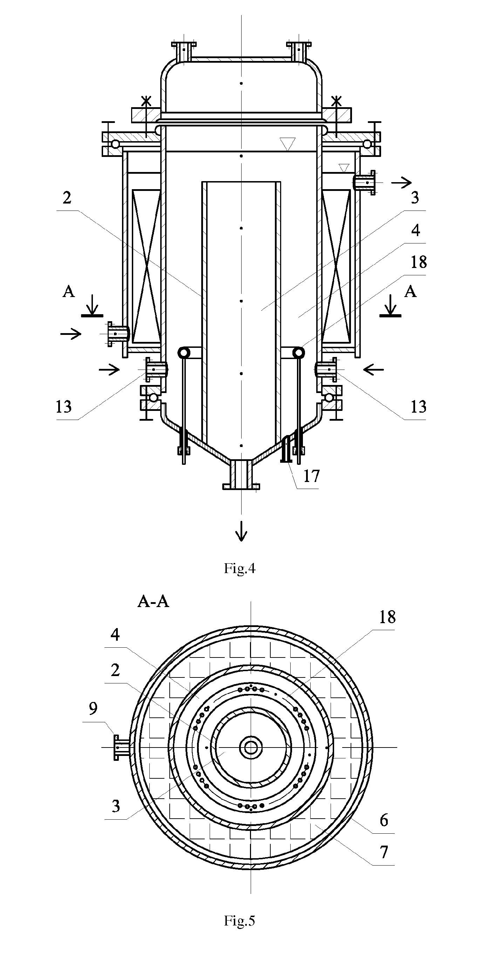

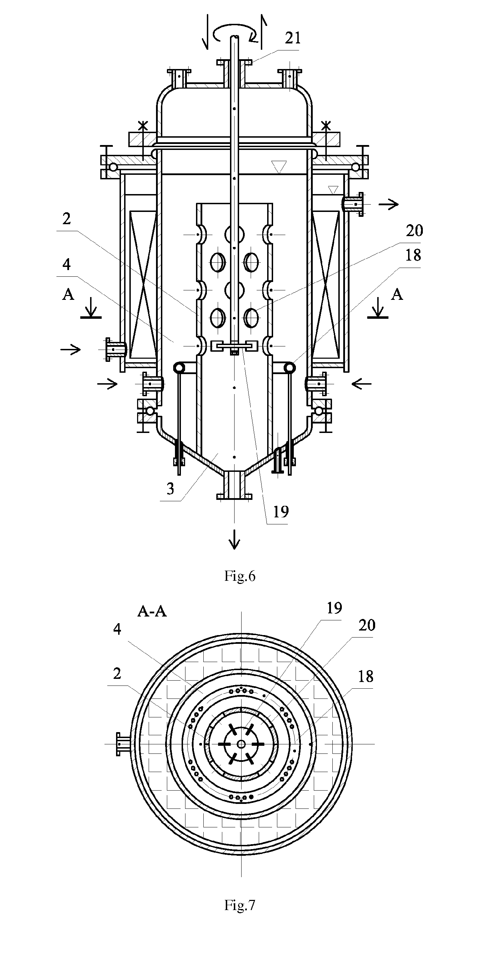

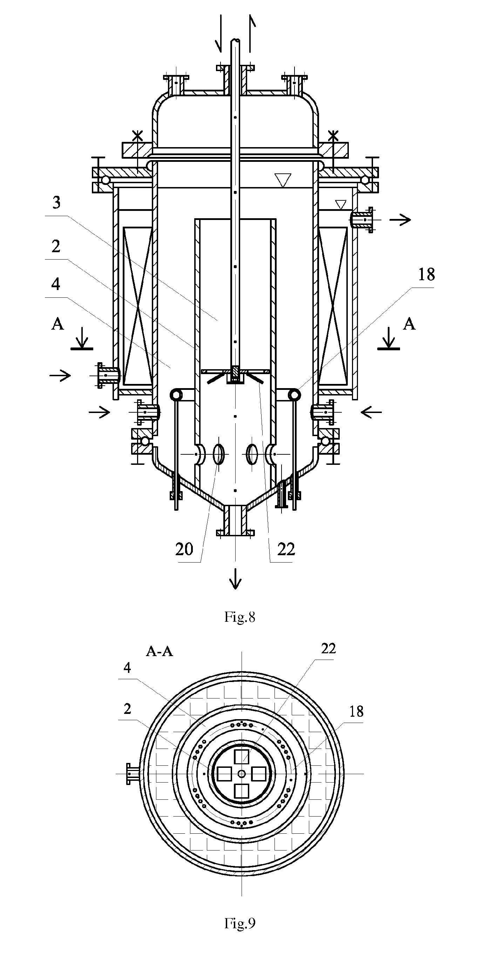

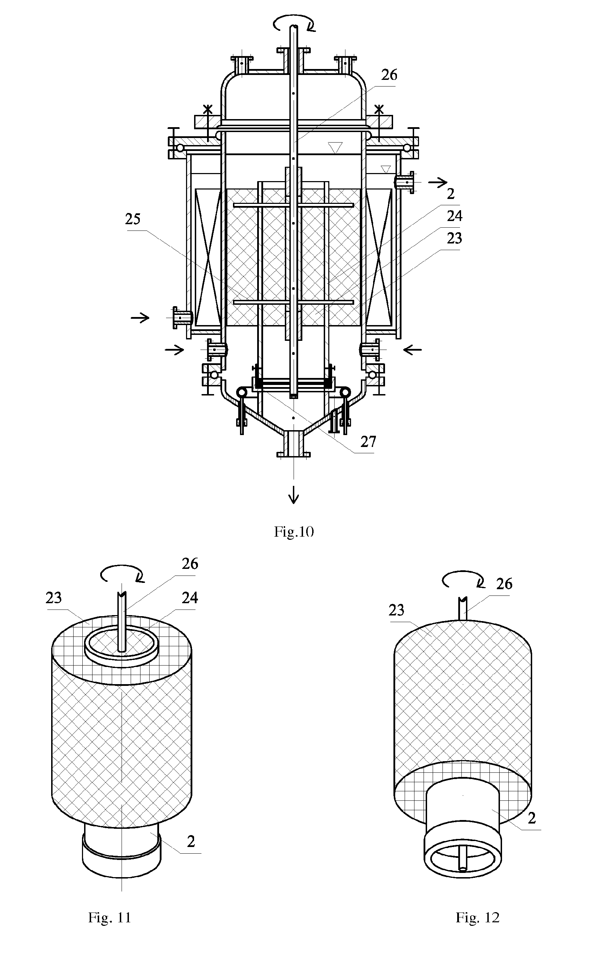

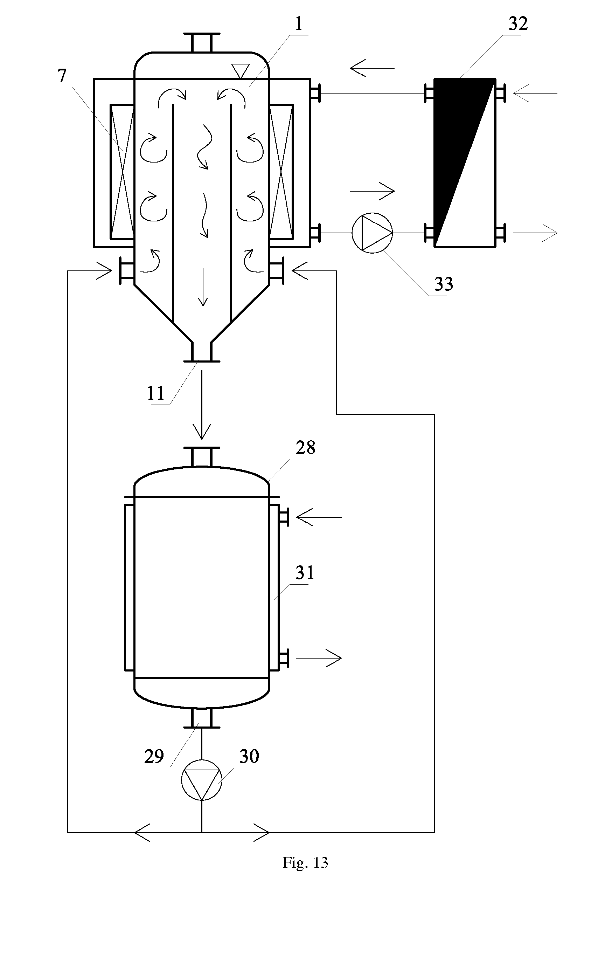

The subjects of the invention are illustrated by examples and in figures in which Fig. 1 shows the dual-chamber reactor in the longitudinal section, Fig. 2 shows the dual-chamber reactor with the gas diffusers in the horizontal pipes filling the annular chamber in the longitudinal section, Fig. 3 shows the reactor from Fig. 2 in cross-section, Fig. 4 shows the dual-chamber reactor with the toroidal gas diffuser filling the annular chamber in the longitudinal section, Fig. 5 shows the reactor from Fig. 4 in cross-section, Fig. 6 shows the dual-chamber reactor equipped with the impeller and the perforation of the cylindrical partition in the upper part, Fig. 7 shows the reactor from Fig. 6 in cross-section, Fig. 8 shows the dual-chamber reactor equipped with the piston and the perforation of the cylindrical partition in the lower part, Fig. 9 shows the reactor from Fig.8 in cross-section, Fig. 10 shows the dual-chamber reactor, wherein in the cylindrical chamber and the annular chamber packings with the material susceptible to magnetic field are supplied, in longitudinal section, Fig. 11 shows the packing of the cylindrical and annular chambers mounted on the shaft in general overview, Fig. 12 shows packing of the cylindrical and annular chambers mounted on the shaft in general bottom view, Fig. 13 shows the sketch of the system with the reactor connected to the heat exchanger and the sump/storage tank.

### Example I (Fig.1)

The reactor 1 for the magnetically assisted processes has the central cylindrical partition 2, which forms the two, cylindrical 3 and annular 4 chambers inside the reactor, attached to the cone-shaped bottom 5. On the reactor housing the outer cylindrical housing 6 is placed, wherein the generator 7 of the rotating magnetic field is positioned between the top cover 8 and the cone-shaped bottom 5. The generator 7 is in the form of the stator of squirrel-cage induction motor and the stator windings are powered by the three-phase current. The outer cylindrical housing 6 has the inlet pipe 9 and the outlet pipe 10 for the thermal medium (in order to supply and to drain the culture medium, ensuring the stable thermal conditions of the rotating magnetic field generator 7). The cone-shaped bottom 5 has the central outlet pipe 11. The top cover 8 has the inlet pipe 12 in order to supply the additional chemical substances into the reactor. Underneath the generator 7 two opposite inlet pipes 13 are connected, in order to supply the reaction medium. The housing of the reactor 1 is connected to the top cover 8 and the cone-shaped bottom 5 by the flange connection. The top cover 8 has the pipe 14 for measuring and controlling apparatus. The medium flows into the reactor through the opposite inlet pipes 13, into the annular chamber 4 and then the medium flows into the cylindrical chamber 3. The medium flows out of the reactor by the central outlet pipe 11.

### Example II (Fig.2,3)

The reactor is made as described in Example I, wherein in the annular chamber 4 above the opposed inlet pipes 13, has six verticals cylindrical partitions 15 in order to produce the additional circulation and/or to increase the residence time distribution of the fluid in the area between the cylindrical chamber 3 and the annular chamber 4. Each of the vertical cylindrical partitions 15 is equipped with the sparger 16 supplying gas to the vertical cylindrical partitions 15. Pipes of sparger 16 are led out through the cone-shaped bottom 5 by pipes. In the cone-shaped bottom 5 of reactor the lateral connector pipe 17 is mounted which allows to remove medium from the annular chamber 4. In the outer cylindrical housing 6 of the reactor, between the generator 7 of the rotating magnetic field and the annular chamber 4, liquid characterizing by the magnetic properties is poured.

### Example III (Fig.4,5)

The reactor is made as described in Example I, wherein in the annular chamber 4, above the opposed inlet pipes 13, the toroidal sparger is mounted 18 in order to supply gas (air). The pipes of the toroidal sparger 18 are led out through the cone-shaped bottom 5 by pipes.

### Example IV (Fig.6,7)

The reactor is made as described in Example III, wherein it is equipped with the turbine impeller 19 positioned in the cylindrical chamber 3 and the upper part of the partition 2 has the perforation 20. The shaft of the turbine impeller 19 is led out through the central pipe 21 mounted in the top cover 8 and the shaft is connected with the drive enabling the reciprocating and/or rotational movement.

### Example V (Fig.8,9)

The reactor is made as described in Example III, wherein it is equipped with reciprocating piston 22 placed in the cylindrical chamber 3 and the lower part of the cylindrical partition 2 has the perforation 20. The piston shaft 22 is led out through the central pipe 21 in the top cover 8 and it is connected with the driver enabling the reciprocating movement.

### Example VI (Fig. 10, 11, 12)

The reactor is made as described in Example I, wherein in the cylindrical chamber 3 and the annular chamber 4 the annular packing 23 and the cylindrical packing 24 are placed and are made from the material which is susceptible to a magnetic field. Packings 23 and 24 are mounted on the rods 25 which are fixed to the shaft 26 and the central cylindrical partition 2. The cylindrical partition 2 is in the form of the two cylinders wherein a slide bearing (motionless slip-ring) 27 is placed.

The movement of catalyst or the immobilized biocatalyst on the rotating packings 23 and 24 allows to improve the operational conditions of the chemical process or in the case of the biochemical process to supply the nutrition efficiently. The annular part of the packing 23 additionally allows to increase the residence time of the air bubblers supplied by means of the located below gas distributor, which increases the interfacial area in the case of the gas-liquid contact.

### Example VII (Fig.13)

The reactor is made as described in Example I, wherein the central outlet pipe 11 of the reactor 1 is connected with the inlet of tank 28 which outlet 29 is connected with the first circulating pump 30 and the tank 28 has the heating jacket 31. The first circulating pump 30 forces the fluid circulation from the tank 28 to the reactor 1, so that the system operates in the closed cycle. This system includes the heat exchanger 32 and the second circulating pump 33 for the medium cooling the rotating magnetic field generator 7. The second circulating pump 33 ensures the flow of the cooling medium in the outer cylindrical housing 6 and the heat exchanger 32. The coolant can be used as the medium applied in the temperature stabilization in the storage tank 28.

## Claims

1. The dual-chamber reactor for magnetically assisted chemical processes involving gases, liquids and solids, and chemical or biochemical reactions carried out by microorganisms is equipped with the two mixing chambers, generator of rotating magnetic field, housing, top cover and bottom with pipes, **characterized in that**, it has the central cylindrical partition (2) that forms the cylindrical (3) and annular (4) chambers inside the reactor (1), fixed to the cone-shaped bottom (5), wherein the reactor has the outer cylindrical housing (6), in which the rotating magnetic field generator (7) is located, between the top cover (8) and the cone-shaped bottom (5), wherein the outer cylindrical housing (6) has the inlet pipe (9) and the outlet pipe (10) of the thermal medium and the cone-shaped bottom (5) has the central outlet pipe (11), the top cover (8) has the inlet pipe (12) in order to supply the additional chemicals into the reactor, and the reactor (1) under generator (7) has at least one inlet pipe (13) in order to supply the reaction medium.

2. The reactor according to claim 1, **characterized in that**, the generator (7) of the rotating magnetic field is made of the stator of squirrel-cage induction motor and the stator windings are powered by the three-phase current.

3. The reactor according to claim 1, **characterized in that**, in the outer cylindrical housing (6) between the generator (7) of the rotating magnetic field and the annular chamber (4) is liquid with the magnetic properties.

4. The reactor according to claim 1, **characterized in that**, in the annular chamber (4), above the inlet pipes (13) at least two vertical cylindrical partitions (15) with the spargers (16) are placed, wherein the vertical partitions (15) are arranged at equal distances to each other in a circle.

5. The reactor according to claim 1, **characterized in that**, in the annular chamber (4) of the reactor, above the outlet pipes (13) has the toroidal sparger (18).

6. The reactor according to claim 5, **characterized in that**, the turbine impeller (19) is placed in the cylindrical chamber (3) and the upper part of the cylindrical partition (2) has the perforations (20), wherein the shaft of impeller (19) is introduced by the central pipe (21) in the top cover (8) and is connected with the drive enabling the reciprocating and/or rotational movement.

7. The reactor according to claim 5, **characterized in that**, has the reciprocating piston (22) placed in the cylindrical chamber (3) and the lower part of the cylindrical partition (2) has the perforation (20), wherein the shaft of the piston (22) is led out through the central outlet pipe (21) in the top cover (8) and is connected with the drive enabling the reciprocating movement.

8. The reactor according to claim 1, **characterized in that**, in the cylindrical chamber (3) and the annular chamber (4) of the reactor the annular packing (23) and the cylindrical packing (24) made from the material which is susceptible to a magnetic field are placed, wherein the packings (23,24) are fixed on the rods (25) to the shaft (26) and the central cylindrical partition (2), which is in the form of the two cylinders, between these cylinders is the slide bearing (27) and the shaft (26) passed through the central pipe (21) in the top cover (8) and is connected with the drive enabling the rotational movement.

9. The reactor according to claim 1, **characterized in that**, the outer cylindrical housing (6) is connected by the inlet (9) and outlet (10) pipes with the heat exchanger (32), wherein between the outlet pipe (10) and the heat exchanger (32) has the second circulation pump (33).

10. The reactor according to claim 1, **characterized in that**, the top cover (8) has the pipe (14) for measuring and controlling apparatus.

11. The reactor according to claim 1, **characterized in that**, the cone-shaped bottom (5) has the lateral connector pipe (17).

12. The reactor system as described in claims 1 to 11, **characterized in that**, the central outlet pipe (11) of the reactor (1) is connected with the inlet to the tank (28) which outlet is connected with the first circulation pump (30), wherein the tank (28) is equipped with the heating jacket (31).
